# EUROPEAN PATENT APPLICATION

(11) **EP 3 821 878 A1**
(43) Date of publication of application: **19.05.2021**
(21) Application number: 20184371.1
(22) Date of filing: 07.07.2020
(51) Int. Cl.: A61K 9/00, A61K 9/20, A23L 2/00

(54) **WATER-SOLUBLE TABLETS, VELOTRANSFER TECHNOLOGY**

(30) Priority: 18.11.2019 IT 201900021360
(71) Applicant: Natural Way Laboratories SRL, 37013 Caprino (VR) (IT)
(72) Inventor: COTTICA, Matteo, 37013 CAPRINO VERONESE (VR) (IT); ACCIARRI, Marta, 37013 CAPRINO VERONESE (VR) (IT); COATTO, Massimo, 37013 CAPRINO VERONESE (VR) (IT); BOCCHI, Maurizio, 37013 CAPRINO VERONESE (VR) (IT)
(74) Representative: Rigamonti, Dorotea

(57) **Abstract**

Object of the present invention is a formulation in tablet form, completely soluble in rapid times both in hot and cold water. The formulation is advantageously used to deliver food supplements.

## Description

### Background

Water-soluble tablets, i.e. formulations that allow rapid dissolution in water, are available.

The fundamental requirements which must be satisfied by said formulations are essentially those of having a rapid and complete dissolution.

Typically, water-soluble tablets have a different behavior depending on the temperature to which they are exposed. In fact, formulations more suitable for hot dissolution and formulations more suitable for cold dissolution are available.

There is a need to have a water-soluble tablet that can advantageously dissolve quickly, for example in less than 30 seconds, and completely in both cold and hot water.

It is an object of the present invention to make available a formulation that satisfies said characteristics.

### Description

The present invention discloses a tablet having a formulation such as to make it rapidly soluble both in hot and cold water. Advantageously, the tablet according to the present invention is used for delivering plant and / or animal extracts such as, by way of non-exhaustive example, plant extracts, mineral salts, vitamins, amino acids, proteins.

In a first embodiment, said formulation comprises, besides the functional active ingredient:
- At least one sweetener, selected from mannitol, xylitol, glucose, sucrose, saccharin, aspartame, sucralose, polydextrose, preferably mannitol 19-24% (w / w) and / or sucralose 0.3-0.6% (w / w) and / or polydextrose 3-6% (w / w);
- At least one binder, selected from gelatins and cellulose derivatives, preferably selected from hydroxypropyl methylcellulose (HPMC), microcrystalline cellulose, arabic gum, glucose syrup and polyethylene glycol, preferably hydroxypropyl methylcellulose 1-5% (w / w) and microcrystalline cellulose 8-13% (w / w);
- At least one disintegrant, selected from maize starch, modified microcrystalline celluloses, polyvinylpyrrolidones, preferably polyvinylpyrrolidone 1-5% (w / w) and / or crospovidone 16-21% (w / w);
- At least one glidant, selected from the magnesium salts of fatty acids 1-5% (w / w);
- At least one anti-caking agent, selected from carbonates, chlorides, silicon dioxide, preferably 1-5% silicon dioxide (w / w);
- At least one preservative, preferably 6-10% citric acid (w / w).

In a preferred embodiment, said formulation comprises, besides the functional active ingredient:
- Mannitol 19-24% (w / w)
- Polydextrose 3-6% (w / w)
- Hydroxypropyl methylcellulose 1-5% (w / w)
- Polyvinylpyrrolidone 1-5% (w / w)
- Microcrystalline cellulose 8-13% (w / w)
- Sucralose 0.3-0.6% (w / w)
- Silicon dioxide 1-5% (w / w)
- Magnesium salts of fatty acids 1-5% (w / w)
- Crospovidone 16-21% (w / w)
- Citric acid 6-10% (w / w).

Said formulation is loaded with an amount of up to 21% (w / w) of a functional active ingredient. In a preferred embodiment, said active ingredient is present in an amount of between 16 and 21% (w / w). Advantageously, said formulation also comprises natural or artificial flavors, preferably in an amount of between 4-8% (w / w).

In a further preferred embodiment, said formulation comprises, besides the functional active ingredient:
- Mannitol 20-23% (w / w)
- Polydextrose 3.5-5% (w / w)
- Hydroxypropyl methylcellulose 2-4% (w / w)
- Polyvinylpyrrolidone 2-4% (w / w)
- Microcrystalline cellulose 9-11% (w / w)
- Sucralose 0.4-0.5% (w / w)
- Silicon dioxide 2-4% (w / w)
- Magnesium salts of fatty acids 1-3% (w / w)
- Crospovidone 16.5-19% (w / w)
- Citric acid 7-9.2% (w / w).

In a preferred embodiment, said formulation comprises, besides the functional active ingredient:
- Mannitol 21.34% (w / w)
- Polydextrose 4.02% (w / w)
- Hydroxypropyl methylcellulose 2.44% (w / w)
- Polyvinylpyrrolidone 2.44% (w / w)
- Microcrystalline cellulose 9.76% (w / w)
- Sucralose 0.49% (w / w)
- Silicon dioxide 2.44% (w / w)
- Magnesium salts of fatty acids 1.71% (w / w)
- Crospovidone 17.07% (w / w)
- Citric acid 8.90% (w / w).

Preferably said formulation further comprises:
- Gliceril behenate E471 (Compritol ® E ATO) 1.95% (w / w)
- Beet juice 3.05% (w / w)

In a preferred embodiment, said formulation further comprises red fruit aroma 3.05% (w / w).

By way of example, the functional active ingredients loaded in the formulation according to the present invention are selected in the group comprising: calcium, magnesium, iron, copper, iodine, zinc, manganese, sodium, potassium, selenium, chromium, molybdenum, fluoride, chloride, phosphorus, and their pharmaceutically acceptable salts; vitamins such as vitamin B1, B2, B6, B12, folic acid, niacin, pantothenic acid, biotin, vitamin H, PP and C, vitamin A, D, E, K, amino acids such as phenylalanine, threonine, tryptophan, methionine, lysine, leucine, isoleucine, valine, histidine, arginine, glycine, glutamine, proline, taurine, cysteine, tyrosine, whey protein, maltodextrin, plant extracts selected from: Abarema C, ABELMOSCHUS ESCULENTUS (L.) MOENCH fructus, ABELMOSCHUS MOSCHATUS MEDIC. Semen, ABIES ALBA MILL. conus, cortex, folium, bud, aetheroleum, resina ABIES BALSAMEA MILL. balsamum ABIES FRASERI LINDL. balsamum ABIES PECTINATA DC. Var. EQUI-TROJANI ASCH. & SINT. ex BOISS bud ABIES SIBIRICA LEDEB. folium ABROMA AUGUSTA L.F. cortex ex radicibus ABRUS PRECATORIUS L. folium, radix ACACIA CATECHU (L.F.) WILLD. gummi, lignum, flos ACACIA DECURRENS WILLD. cortex, flos, gummi ACACIA FARNESIANA WILLD. flos, gummi ACACIA LAETA R. BR. ex BENTH.. gummi ACACIA NILOTICA WILLD EX DEL. (syn. ACACIA VERA WILLD.) gummi ACACIA SENEGAL. WILLD. cortex, gummi ACACIA SEYAL DELILE gummi ACACIA SUMA KURZ. gummi, lignum ACALYPHA INDICA L. folium, radix ACANTHUS MOLLIS L. folium, herba, radix ACER CAMPESTRIS L. bud ACER NEGUNDO L. cortex ACER SACCHARINUM L. cortex, lignum ACHILLEA AGERATUM L. herba c. floribus ACHILLEA ATRATA L. herba c. floribus ACHILLEA ERBA-ROTA ALL. herba ACHILLEA ERBA-ROTA SSP MOSCHATA RICH. herba c. floribus, capitula, oleum ACHILLEA MILLEFOLIUM L. herba c. floribus, capitula, aetheroleum ACHILLEA NANA L. herba c. floribus ACHILLEA PTARMICA L. radix, herba c. floribus, capitula ACHRAS SAPOTA L. flos ACTINIDIA CHINENSIS PLANCH. (syn DELICIOSA-KIWI) fructus, bud ACTINOMERIS HELIANTHOIDES (MICHX.) NUTT. radix ADANSONIA DIGITATA L. cortex, folium, radix, semen, fructus ADHATODA VASICA NEES flos, folium, radix ADIANTUM CAPILLUS-VENERIS L. folium, herba ADIANTUM PEDATUM L. folium, herba ADOXA MOSCHATELLINA L. herba AEGLE MARMELOS CORREA cortex, folium, radix, semen AEGOPODIUM PODAGRARIA L. herba AESCULUS HIPPOCASTANUM L. cortex, flos, folium, bud AFRAMOMUM EXSCAPUM HEPPER semen AFRAMOMUM MELEGUETA K. SCHUM. Semen, AGARICUS BLAZEI sporophorum AGAVE AMERICANA L. folium, radix AGAVE SISALANA PERR. folium, radix AGAVE TEQUILANA F.A.C. WEBER folium AGRIMONIA EUPATORIA L. folium, herba, summitas AGRIMONIA ODORATA AUCT. folium, herba, summitas AGROPYRON REPENS BEAUV. rhizoma AJUGA CHAMAEPITYS SCHREB. flos, folium, herba c. floribus AJUGA IVA SCHREB. summitas AJUGA REPTANS L. folium, herba c. floribus ALBIZIA ANTHELMINTICA BRONGN. cortex ALCHEMILLA ALPINA L. herba ALCHEMILLA VULGARIS L. s.l. folium, herba, radix ALETRIS FARINOSA L. bulbus, radix ALISMA ORIENTALE JUZEP. rhizoma ALISMA PLANTAGO AQUATICA L. rhizoma, folium ALLIARIA PETIOLATA C. ET G. herba, semen ALLIUM ASCALONICUM L. bulbus, herba ALLIUM CEPA L. bulbus ALLIUM PORRUM L. bulbus, herba ALLIUM SATIVUM L. bulbus ALLIUM SCHOENOPRASUM L. herba ALLIUM URSINUM L. bulbus, folium ALNUS GLUTINOSA GAERTN. cortex, folium, bud ALNUS INCANA (L.) MOENCH bud ALOE AFRICANA MILL. succus, gel sine cute ALOE ARBORESCENS MILL. succus, gel sine cute ALOE FEROX MILL. succus, gel sine cute ALOE PERRYI BAK. succus, gel sine cute ALOE PLICATILIS MILL. succus, gel sine cute ALOE VERA L. (Syn. ALOE BARBADENSIS MILL.) succus, gel sine cute ALPINIA GALANGA WILLD. rhizoma ALPINIA OFFICINARUM HANCE rhizoma ALSIDIUM HELMINTHOCHORTON K. thallus ALTHAEA OFFICINALIS L. flos, folium, radix ALTHAEA ROSEA L. flos AMARANTHUS CAUDATUS L. fructus, semen AMARANTHUS CRUENTUS L. fructus, semen AMARANTHUS HYPOCONDRIACUS L. fructus, semen AMOMUM XANTHIODES WALLICH semen AMORPHOPHALLUS KONJAC KOCH tuber, amylum AMPELOPSIS VEITCHII HORT (syn. PARTHENOCISSUS TRICUSPIDATA (SIEBOLD & ZUCC.) PLANCH.) bud, young shoots AMYGDALUS COMMUNIS L. semen, oleum, bud AMYRIS BALSAMIFERA L. lignum ANACARDIUM OCCIDENTALE L. folium, fructus, radix, semen, pedunculus ANACYCLUS PIRETHRUM LINK radix ANANAS COMOSUS (L.) MERR. stipites, fructus ANCHIETEA SALUTARIS ST. HILL. radix ANDROGRAPHIS PANICULATA NEES folium, herba, radix, summitas ANEMOPAEGMA MIRANDUM MART EX DC (Catuaba) cortex, ANETUM GRAVEOLENS L. semen ANGELICA ARCHANGELICA L. folium, fructus, radix, herba, aetheroleum ANGELICA SINENSIS DIELS. fructus, radix ANGELICA SYLVESTRIS L fructus, herba, radix, summitas ANIBA ROSAEODORA DUCKE lignum, aetheroleum ANNONA MURICATA L. fructus ANNONA RETICULATA L. fructus ANNONA SQUAMOSA L. fructus ANOGEISSUS LATIFOLIA WALL. gummi ANREDERA BASELLOIDES BAILE tuber ANTENNARIA DIOICA GAERTN. capitula, herba ANTHEMIS COTULA L. herba c. floribus ANTHEMIS TINCTORIA L. capitula, herba c. floribus, radix ANTHRISCUS CEREFOLIUM HOFFM. summitas c. floribus ANTHRISCUS SYLVESTRIS HOFFM. fructus, herba ANTHYLLIS VULNERARIA L. flos, herba ANTIRRHINUM MAJUS L. herba APHANES ARVENSIS L. folium, herba c. floribus APHANIZOMENON FLOS AQUAE (Alga klamath) bacteria APIUM GRAVEOLENS L. fructus, summitas, folium, radix, APIUM GRAVEOLENS L. var. RAPACEUM (MILL.) GAUDIN radix, folium, latex APIUM GRAVEOLENS L. var. SECCALINUM folium AQUILARIA AGALLOCHA ROXB. lignum AQUILARIA MALACCENSIS LAM. lignum ARACHIS HYPOGAEA L. semen ARALIA RACEMOSA L. radix ARBUTUS UNEDO L. flos, folium, radix ARCTIUM LAPPA L. fructus, herba, radix, oleum ARCTIUM MINUS BERNH. folium, fructus, radix ARCTIUM TOMENTOSUM MILL radix ARCTOSTAPHYLOS UVA-URSI (L.) SPRENG. folium ARMORACIA RUSTICANA GAERNT radix ARONIA MELANOCARPA (MICHX.) ELLIOT fructus ARTEMISIA ABROTANUM L. folium, herba, summitas ARTEMISIA ABSINTHIUM L. capitula, herba c. floribus, folium ARTEMISIA CAMPESTRIS L. capitula, herba, folium, summitas ARTEMISIA CAPILLARIS THUNB. herba ARTEMISIA CHINENSIS L. folium ARTEMISIA DRACUNCULUS L. folium, herba c. floribus ARTEMISIA FRIGIDA WILLD. herba, summitas ARTEMISIA GENIPI WEBER flos, herba c. floribus, summitas ARTEMISIA GLACIALIS L. herba c. floribus, summitas ARTEMISIA JUDAJCA LOUR. herba c. floribus ARTEMISIA MARITIMA BERTOL. herba c. floribus, folium, summitas ARTEMISIA NANA GAUD. herba c. floribus ARTEMISIA PALLENS WALL EX OC herba c. floribus, summitas ARTEMISIA PONTICA L. herba c. floribus, ARTEMISIA PROCERA WILLD. (syn ARTEMISIA ABROTANUM L.) herba c. floribus, summitas ARTEMISIA UMBELLIFORMIS LAM. herba c. floribus ARTEMISIA VALLESIACA ALL. herba c. floribus, summitas ARTEMISIA VERLOTORUM LAMOT. herba c. floribus ARTEMISIA VULGARIS L capitula, herba c. floribus, radix, folium, rhizoma ARTOCARPUS ALTILIS FOSBERG lignum, semen, fructus ASCOPHYLLUM NODOSUM DE JOLIS thallus ASIMINIA TRILOBA DUNAL fructus ASPALATHUS LINEARIS (N.L. BURM.) R.DAHLGR. (Rooibos) folium, herba ASPARAGUS OFFICINALIS L. radix, rhizoma ASPARAGUS RACEMOSUS (WILLD.) OBERM. radix ASPLENIUM ADIANTUM NIGRUM L. folium ASPLENIUM RUTA MURARIA L. herba ASPLENIUM TRICHOMANES L. folium ASTRAGALUS ADSCENDENS B. ET H. gummi ASTRAGALUS CRETICUS LAM. gummi ASTRAGALUS GUMMIFER LABILL gummi ASTRAGALUS MEMBRANACEUS BUNG. radix ASTRAGALUS MICROCEPHALUS WILLD. radix ASTRAGALUS VERUS OLIV. gummi ASTRANTIA MAJOR L. radix ATHAMANTHA CRETENSIS L. flos, folium, fructus, herba, summitas ATHAMANTHA MACEDONICA SPR. fructus, herba ATHEROSPERMA MOSCHATUM LABIL. cortex, folium AURICULARIA AURICULA-JUDAE (BULL.: FR.) WETTST. sporophorum AVENA FATUA L. fructus, summitas AVENA SATIVA L. fructus, summitas AVERRHOA CARAMBOLA L. folium, fructus BACOPA MONNIERI (L.) PENNELL herba, summitas, folium, rhizoma BACTRIS GASIPAES KUNTH fructus, medulla ex bud BALANITES AEGYPTIACA DEL. cortex, folium, fructus, semen BALLOTA NIGRA L. SSP FOETIDA HAYEK herba, herba c. floribus, folium BAMBUSA VULGARIS SCHRAD. Ex. J.C. WENDL. sùrculi (giovane fusto), essudato BAMBUSA spp germen, sùrculi (giovane fusto), essudato BAPTISIA TINCTORIA R. BR. radix BARBAREA VERNA (P.MILL.) ASCHERS. folium, oleum, semen BARBAREA VULGARIS R. BR. herba BAROSMA BETULINA BART ET WEN folium BAROSMA CRENULATA HOOK folium BAROSMA SERRATIFOLIA WILLD. folium BELLIS PERENNIS L. capitula, folium BERBERIS ARISTATA DC cortex arboris BERBERIS VULGARIS L. cortex ex radicibus, folium, semen BERGENIA CRASSIFOLIA FRITSCH folium BERTHOLLETIA EXCELSA HUMB. ET BONPL. semen, oleum, BETA VULGARIS L. radix, herba, folium BETA VULGARIS L. ssp VULGARIS radix, folium BETULA LENTA L. cortex, folium, oleum BETULA PENDULA ROTH. cortex, folium, bud, resina, semen, lympha, flores (amenti) BETULA PUBESCENS EHRH. folium, bud, flores (amenti), lympha BIDENS TRIPARTITA L. herba BIGNONIA QUINQUEFOLIA L. folium BIXA ORELLANA L. semen BORONIA MEGASTIGMA NEES herba BORRAGO OFFICINALIS L. semen, oleum BOSWELLIA CARTERII BIRDW. resina, gummi BOSWELLIA SERRATA ROXB. resina, gummi BOVISTA PLUMBEA PERS. sporophorum BOWDICHIA VIRGILIOIOES H.B.K. cortex BRACHYCLADUS STUCKERTI SP. herba, radix BRASSICA JUNCEA (L.) CZERN. var. RUGOSA (ROXB.) N. TSEN et S. N. LEE semen BRASSICA NAPUS L. semen BRASSICA NAPUS L. var. NAPOBRASSICA MILL. radix BRASSICA NIGRA (L.) KOCH. semen BRASSICA OLERACEA L. convar. ACEPHALA (DC) ALEF folium BRASSICA OLERACEA L. convar. ACEPHALA (DC) ALEF var. GONGYLOIDES L. folium BRASSICA OLERACEA L. convar. CAPITATA (L.) ALEF. folium BRASSICA OLERACEA L. var. BOTRYTIS L. folium, inflorescentia BRASSICA OLERACEA L. var. GEMMIFERA folium BRASSICA OLERACEA L. var. ITALICA folium, inflorescentia BRASSICA PEKINENSI (LOUR.) RUPR. folium BRASSICA RAPA L. radix, folium, latex BRASSICA RAPA L. ssp. BRASSICA RAPA L. var. NIPPONICA folium, germen BRUCEA FERRUGINEA L'HERIR. cortex BRUCEA JAVANICA MERR. fructus BUPLEURUM ROTUNDIFOLIUM L. herba BURSERA GUMMIFERA ENGL. resina, lignum BURSERA TOMENTOSA TRIA ET P. resina, lignum BUTOMUS UMBELLATUS L. folium, rhizoma CAESALPINIA BONDUC ROXB. folium, radix, semen CAESALPINIA BREVIFOLIA ROXB. fructus CAESALPINIA ECHINATA LAMK. lignum CAKILE MARITIMA SCOP. herba CALAMINTHA NEPETA SAVI SSP. GLANDULOSA BALL. folium, summitas CALENDULA ARVENSIS L. capitula CALENDULA OFFICINALIS L. capitula, ligula, herba CALLIANDRA HOUSTONI BENTH. cortex ex radicibus CALLUNA VULGARIS HULL. flos, herba c. floribus, summitas, bud, cauliculi (young shoots) CAMELINA SATIVA L. CRANTZ semen, oleum CAMELLIA SINENSIS (L.) KUNTZE folium CAMPHOROSMA MONSPELIACA L. summitas, CANAGA ODORATA HOOK F. ET TH. (YLANGYLANG) flos, aetheroleum CANARIUM LUZONICUM A. GRA Y resina, semen CANARIUM ROSTRATUM ZIPPEL resina CANAVALIA ENSIFORMIS (L.) DC. fructus CANNABIS SATIVA L. semen, oleum CAPPARIS SPINOSA L. fructus, flos CAPSELLA BURSA-PASTORIS MED herba CAPSICUM ANNUUM var. GROSSUM (L.) SENDTN. oleoresina, fructus CAPSICUM ANNUUM var. LONGUM Sendtn. oleoresina, fructus CAPSICUM FRUTESCENS L. oleoresina, fructus CARBO VEGETABILIS ex LIGNO pulvis CAREX ARENARIA L . rhizoma CARICA PAPAYA L. fructus, semen, folium CARISSA CARANDAS L . cortex, lignum, radix CARLINA ACAULIS L. radix CARLINA ACAULIS ssp. SIMPLES NYMAN radix CARPINUS BETULUS L. folium, bud CARTHAMUS LANATUS L. flos, semen CARTHAMUS TINCTORIUS L. flos, semen, oleum CARUM CARVI L. fructus, aetheroleum CARYA ALBA NUTT. fructus, lignum CARYA ILLINOENSIS (WANGENH.) K.KOCH. fructus CARYOPHYLLUS AROMATICUS L. fructus, flos, folium, aetheroleum CASCARILLA MACROCARPA WEDD. cortex CASCARILLA MAGNIFOLIA WEDD. cortex CASSIA ACUTIFOLIA DEL. folium, fructus CASSIA ANGUSTIFOLIA VAHL. folium, fructus CASSIA FISTULA L. pulpa fructi, fructus CASSIA ITALICA F.W. ANDR. folium CASSIA MIMOSOIDES L. var. NOMAME MAKINO folium, fructus CASSIA OCCIDENTALIS L. folium, radix CASSIA SENNA L. folium, fructus CASSIA TORA L. s.l. folium, semen, bud CASTANEA SATIVA MILL. (syn. Castanea vesca Gaertn.) folium, semen, bud CASTILLOA ELASTICA CERV. latex, resina CATALPA BIGNONIOIDES WALT. folium CEANOTHUS AMERICANUS L. cortex, radix, folium CECROPIA PELTATA L. folium CEDRUS LIBANI A. RICH. bud, aetheroleum, sùrculi (young shoots) CEIBA PENTANDRA GAERTN. semen CENTAUREA BEHEN L. radix CENTAUREA CALCITRAPA L. capitula, herba, flos, herba c. floribus, radix, folium CENTAUREA CYANUS L. flos CENTAUREA JACEA L. herba c. floribus CENTAUREA MONTANA L. capitula CENTAURIUM ERYTHRAEA RAFN. summitas CENTELLA ASIATICA L. herba, folium CENTRANTHUS RUBER DC radix, CERATONIA SILIQUA L. fructus CERCIS SILIQUASTRUM L. bud, cortex CEREUS GRANDIFLORUS MILL. flos CETERACH OFFICINARUM DC folium, rhizoma CETRARIA ISLANDICA ACH. thallus CHAENOMELES SPECIOSA (SWEET) NAKAI fructus CHAMAEMELUM NOBILE (L.) ALL. (Syn ANTHEMIS NOBILIS L.) herba, aetheroleum CHELONE GLABRA L. herba c. floribus CHENOPODIUM BOTRYS L. herba, herba c. floribus CHENOPODIUM QUINOA WILLD. fructus, semen CHENOPODIUM VULVARIA L. herba, folium CHIMAPHILA UMBELLATA BARTON cortex, herba, radix, folium, summitas CHIOCOCCA ALBA HITCH. radix CHIONANTHUS VIRGINICA L. cortex ex radicibus CHLORELLA PYRENOIDISA CHICK thallus CHLORELLA VULGARIS SPECIES thallus CHLOROSTIGMA STUCKERTIANUM K. radix, folium CHONDRUS CRISPUS STACKHOUSE thallus CHRYSANTELLUM AMERICANUM VATKE herba CHRYSANTHEMUM BALSAMITA DESF. capitula, herba, folium CHRYSOPHYLLUM CAIMITO L. fructus CIBOTIUM BAROMETZ (L.) J. SM. folium CICHORIUM ENDIVA L. folium CICHORIUM INTYBUS L. summitas, radix CICHORIUM INTYBUS L. var. FOLIOSUM HEGI folium CIMICIFUGA RACEMOSA NUTT. Rhizoma, CINCHONA CALISAYA WEDD. cortex CINCHONA CORDIFOLIA MUT. cortex CINCHONA LANCIFOLIA MUT. cortex CINCHONA LEDGERIANA MOENS-T. cortex CINCHONA MICRANTHA E.et P. cortex CINCHONA NITIDA R. et P. cortex CINCHONA OFFICINALIS L. cortex CINCHONA PERUVIANA HOW. cortex CINCHONA PITAYENSIS WEDD. cortex CINCHONA SUCCIRUBRA PAV. cortex CINNAMOMUM CAMPHORA (L.) J. PRESL cortex, folium, aetheroleum CINNAMOMUM ZEYLANICUM BLUME. cortex, aetheroleum CIRSIUM ARVENSE SCOP. herba, radix CISSUS ALATA JACQ. herba, folium CISTANCHE SALSA (C. A. MEY.) BECK caulis CISTUS INCANUS L. gummi, radix, folium, herba CISTUS LADANIFERUS L. gummi CISTUS MONSPELLIENSIS L. gummi CITRULLUS LANATUS (THUNB.) MATSUM. Et NAKAI var. LANATUS fructus CITRUS AURANTIFOLIA SWINGLE fructus, CITRUS AURANTIUM L. subsp. AMARA fructus, fructus immaturi, pericarpium, aetheroleum, aetheroleum ex floribus, flos, CITRUS BERGAMIA RISSO & POIT. succus CITRUS LIMON BURM. F. pericarpium, aetheroleum, cortex ramorum (scorza dei rami) CITRUS MAXIMA (BURM.) MERR. (syn. CITRUS DECUMANA L. /CITRUS GRANDIS OSBECK) pericarpium, folium, flos, semen CITRUS NOBILIS LOUR. pericarpium, aetheroleum CITRUS RETICULATA L. pericarpium, aetheroleum CITRUS SINENSIS L. var. DULCIS pericarpium, aetheroleum, folium, flos, aetheroleum ex floribus CITRUS x PARADISI MACFAD. pericapium, semen CLADONIA PYXIDATA FR. thallus CLADONIA RANGIFERINA WEB. thallus CLITORIA TERNATEA L. radix, semen CNICUS BENEDICTUS L. folium, fructus, herba c. floribus, summitas COCHLEARIA OFFICINALIS L. folium, herba c. floribus COCOS NUCIFERA L oleum, fructus CODONOPSIS PILOSULA NANNFELD flos, herba, radix COFFEA ARABICA L. semen COIX LACRYMA-JOBI L. semen COLA ACUMINATA SCHOTT et ENDL. semen COLA NITIDA SCHOTT et ENDL. semen COLLINSONIA CANADENSIS L. radix COMBRETUM MICRANTHUM DON. folium COMBRETUM SUNDAICUM MIQ. folium, herba COMMELINA TUBEROSA L. herba COMMIPHORA ABYSSINICA ENGL. resina, gummi COMMIPHORA AFRICANA ENGL. gummi COMMIPHORA MOLMOL ENGL. gummi COMMIPHORA MUKUL HOOK oleum-gummi-resina COMMIPHORA MYRRHA ENGL. oleum-gummi-resina COMMIPHORA OPOBALSAMUM ENGL. oleum-gummi-resina, lignum, fructus COMMIPHORA SCHIMPERI ENGL. oleum-gummi-resina CONVOLVULUS ARVENSIS L. herba, folium CONYZA CANADENSIS (L.) CRONQ. (Sy. Erygeron canadensis) herba, summitas COPAIFERA GUYANENSIS DESF. balsamum COPAIFERA LANGSDORFII DESF. balsamum COPTIS JAPONICA MAKINO radix, COPTIS TEETA WALL. rhizoma COPTIS TRIFOLIA SALISB. rhizoma CORALLINA OFFICINALIS L. thallus CORDIA MYXA L. fructus CORDYCEPS SINENSIS (syn. Paecilomyces hepiali Chen) sporophorum, micelium CORIANDRUM SATIVUM L. fructus CORNUS FLORIDA L. cortex ex radicibus CORNUS MAS L. CORNUS OFFICINALIS SIEB. ET ZUCC. fructus CORNUS SANGUINEA L. fructus, bud CORONOPUS SQUAMATUS ASCHERS. herba CORRIGIOLA TELEPHIIFOLIA POURR. radix CORYLUS AVELLANA L. fructus, bud COSCINIUM FENESTRATUM COLIBR. rhizoma COSTUS SPECIOSUS SM. radix COTINUS COGGYGRIA SCOP. cortex CRAMBE MARITIMA L. folium, fructus CRATAEGUS AZAROLUS L. flos, folium CRATAEGUS CURVISEPALA LIND. flos, folium CRATAEGUS LAEVIGATA DC flos, folium CRATAEGUS MONOGYNA JACQ. flos, folium, sùrculi (young shoots) CRATAEGUS NIGRA WALD. ET KIT. flos, flos c. foliis, folium CRATAEGUS OXYACANTHA MEDICUS flos, folium, sùrculi (young shoots), fructus CRATAEGUS PENTAGYNA W. ET K. flos, flos c. foliis, folium CRESCENTIA CUJETE L. fructus CRITHMUM MARITIMUM L. herba CROCUS SATIVUS L. stigmata CROTON ELUTERIA SW. cortex CUCUMIS MELO L. fructus CUCUMIS SATIVUS L. fructus CUCURBITA MAXIMA DUCHESNE fructus, semen, flos, oleum CUCURBITA PEPO L. fructus, flos CUCURBITA PEPO L. var. MELOPEPO (L.) HARZ. fructus, flos CUCURBITA PEPO L. var. OLEIFERA PIETSCH semen CUMINUM CYMINUM L. semen, aetheroleum CUPRESSUS SEMPERVIRENS L. conus, lignum, folium, galbuli, aetheroleum ex galbuli CURCUMA DOMESTICA VALETON (syn CURCUMA LONGA L.) rhizoma CURCUMA XANTHORRHIZA ROXB. rhizoma CURCUMA ZEDOARIA ROSC. rhizoma CUSCUTA EPITHYMUS L. herba CUSCUTA EUROPAEA L. herba CUSPARIA OFFICINALIS HANC. cortex CUSPARIA TRIFOLIATA ENG. cortex CYAMOPSIS TETRAGONOLOBA (L.) TAUB. (GUAR) semen CYCLANTERA PEDATA SCHARD (Caigua) fructus CYDONIA OBLONGA MILL. fructus CYMBOPOGON CITRATUS STAPF. herba, folium, rhizoma, summitas, oleum CYMBOPOGON FLEXUOSUS STAPF. herba, summitas CYMBOPOGON LANIGER PERR. Herba, CYMBOPOGON MARTINI WATS herba CYMBOPOGON NARDUS RENDL. herba, aetheroleum, folium, summitas CYMBOPOGON SCHOENANTHUS (L.) SPRENG. var. MOTIA herba CYMBOPOGON WINTERIANUS JOW. herba, aetheroleum CYNARA CARDUNCULUS L. capitula, folium CYNARA SCOLYMUS L. folium, capitula CYNODON DACTYLON PERS. rhizoma CYPERUS LONGUS L. radix CYPERUS ROTUNDUS L. radix CYPRIPEDIUM PUBESCENS WILLD. rhizoma, radix CYTINUS HYPOCISTIS L. succus inspissitus DACTYLIS GLOMERATA L. fructus, herba, summitas DAEMONOROPS DRACO BLUME fructus, resina DAEMONOROPS JENKINSIANA (GRIFF.) MART. germen DAEMONOROPS PROPINQUUS BECC. resina DAHLIA PINNATA CAV. (syn DAHLIA VARIABILIS (WILLD.) DESF.) radix DANAIS FRAGRANS COMMERS cortex ex radicibus, cortex arboris DAUCUS CAROTA L. radix DAVILLA RUGOSA POIR. folium DESCURAINIA SOPHIA WEBB. semen, herba DESMODIUM ADSCENDENS (Sw.) D.C. folium DIANTHUS CARYOPHYLLUS L. flos DICENTRA CANADENSIS WALD. tuber DICENTRA CUCULLARIA BERNH. tuber DICYPELLIUM CARYOPHYLLATUM (MART.) NEES. cortex, lignum DIOSCOREA ALATA L. tuber DIOSCOREA COMPOSITA HEMSL. rhizoma DIOSCOREA OPPOSITA THUM. radix, rhizoma DIOSCOREA VILLOSA L. radix, rhizoma DIOSPYROS KAKI L. f. fructus DIOSPYROS VIRGINIANA L. fructus DIOTIS CANDIDISSIMA DESF. herba DIPLOTAXIS ssp. herba DIPLOTAXIS TENUIFOLIA DC herba DIPSACUS FULLONUM L. radix DIPTEROCARPUS ALATUS ROXB. balsamum DOREMA AMMONIACUM D. DON. gummi, resina DORONICUM PARDALIANCHES L. herba, radix DORONICUM PLANTAGINEUM L. herba, radix DORSTENIA CONTRAJERVA L. radix DRACOCEPHALUM MOLDAVICA L. herba c. floribus DRACUNCULUS VULGARIS SCHOTT. (syn. ARUM DRACUNCULUS L.) rhizoma DRIMYS WINTERI FORST. cortex arboris DROSERA ANGLICA HUDSON herba DROSERA INTERMEDIA HAYNE herba DROSERA PELTATA THUNB. herba DROSERA RAMENTACEA BURCH herba DROSERA ROTUNDIFOLIA L. herba c. floribus DUNALIELLA SALINA E.C. TEODORESCO thallus DURIO ZIBETHINUS L. fructus, DURVILLEA ANTARTICA (CHAMISSO) HARIOT syn FUCUS ANTARTICUS CHAMISSO thallus DYSOXYLUM LOUREIRI PIERRE aetheroleum ECHINACEA ANGUSTIFOLIA DC . herba, radix ECHINACEA PALLIDA BRITTON herba, radix ECHINACEA PURPUREA MOENCH. herba, radix ECHIUM PLANTAGINEUM L. oleum EISENIA BICYCLIS (KJELLMAN) SETCHELL (syn. ECKLONIA BICYCLIS KIELLMAN) thallus ELAEIS GUINEENSIS JACQ. fructus, oleum ELETTARIA CARDAMOMUM WHITE et MASON semen, aetheroleum ELEUTHEROCOCCUS SENTICOSUS MAXIM. radix ELYMUS REPENS GOULD. rhizoma ENTEROMORPHA spp. thallus EPILOBIUM ANGUSTIFOLIUM L. herba EPILOBIUM PARVIFLORUM SCHERB. herba EQUISETUM ARVENSE L. herba, bud EQUISETUM FLUVIATILE L. herba EQUISETUM HYEMALE L. herba EQUISETUM TELMATEJA EHRH. herba ERICA CINEREA L. flos, folium c. floribus ERIGERON CANADENSIS L. (syn. CONYZA CANADENSIS (L.) CRONQUIST) herba ERIOBOTRYA JAPONICA (THUNB.) LINDL. fructus ERIODICTYON CALIFORNICUM TOR. herba, folium, summitas ERODIUM CICUTARIUM L. HÉRIT. herba, radix, summitas c. floribus ERODIUM MOSCHATUM L. HÉRIT. herba ERUCA SATIVA MILL. herba, folium ERYNGIUM CAMPESTRE L. herba, radix ERYNGIUM PRAEALTUM A. GRAY radix ERYTHRAEA CENTAURIUM L. (syn. CENTAURIUM ERYTHRAEA RAFN.) herba c. floribus, summitas ERYTHRAEA CHILENSIS PERS. herba c. floribus ERYTHRAEA SPICATA PERS. herba c. floribus ERYTHROXYLUM CATUABA A. J. SILVA ex RAYM.-HAMET cortex ESCHSCHOLTZIA CALIFORNICA CHAM. herba, summitas, flos, radix EUCALYPTUS CITRIODORA HOOK cortex, flos, folium, lignum, aetheroleum EUCALYPTUS GLOBULUS LABILL. flos, fructus, folium, lignum, aetheroleum EUCALYPTUS ODORATA B. et SCH. folium, lignum EUCALYPTUS SMITHII BAKER folium, lignum, aetheroleum EUCARYA SPICATA SPRAGUE-SUM. lignum, aetheroleum EUCHEUMA HORRIDUM AGARDH. thallus EUCHEUMA SPICIFORME AGARDH. thallus EUCHEUMA SPINOSUM AGARDH. thallus EUCOMMIA ULMOIDES OLIVIER aubier, cortex EUGENIA CHEQUEN MOL. herba, folium EUGENIA UNIFLORA L. cortex, flos, folium EUPHORIA LONGANA LAM. semen EUPHRASIA OFFICINALIS L.s.l. herba c. floribus EUPHRASIA STRICTA D. WOLF. herba c. floribus EUTERPE OLERACEA MART. germen, fructus EXOSTEMA CARIBAEUM SCHULT. Cortex, EXOSTEMA FLORIBUNDUM ROEM-S. cortex FABIANA IMBRICATA R. et P. cortex, folium, herba, lignum FAGOPYRUM ESCULENTUM MOENCH fructus, folium, flores FAGUS SYLVATICA L. pix, bud FERULA ASSA FOETIDA L. gummi, radix FERULA GALBANIFLUA BOISS-B. gummi, resina FERULA NARTHEX BOISS-B. gummi, resina FERULA PERSICA WILLD. gummi, resina FERULA SUMBUL HOOK F. radix FICUS ANTHELMINTICA MART. latex FICUS BENGHALENSIS L. cortex, fructus, latex FICUS CARICA L. folium, bud, latex FICUS CYSTOPODA MIQ. cortex arboris FICUS ELASTICA ROXB. latex FICUS RELIGIOSA L. cortex, folium, resina, semen FILIPENDULA ULMARIA MAX. flos, folium, herba, radix, summitas FILIPENDULA VULGARIS MOENCH. flos, folium, herba, radix, summitas FOENICULUM VULGARE MILL. fructus, aetheroleum FOMES FOMENTARIUS FR. sporophorum FOMES IGNARIUS L. sporophorum FORTUNELLA spp. fructus FRAGARIA x ANANASSA DUCHESNE ex ROZIER fructus FRAGRARIA VESCA L. fructus, folium FRAXINUS EXCELSIORIUS L. cortex, folium, bud FRAXINUS ORNUS L. folium, manna FUCUS SERRATUS L. thallus FUCUS VESICULOSUS L. thallus FUMARIA OFFICINALIS L. folium, herba c. floribus, summitas GALEGA OFFICINALIS L folium, herba c. floribus GALEOPSIS SEGETUM NECKER herba c. floribus, summitas GALIPEA CUSPARIA L. cortex GALIUM APARINE L. flos, herba c. floribus GALIUM CRUCIATA SCOP. flos, herba c. floribus GALIUM MOLLUGO L. summitas GALIUM ODORATUM SCOP. folium, herba c. floribus, summitas GALIUM VERUM L. flos, herba c. floribus, summitas GANODERMA LUCIDUM (CURTIS) P. KARST. (RHEISHI) sporophorum GARCINIA CAMBOGIA (GAERNT) DESR. fructus, gummi-resina GARCINIA MANGOSTANA L. pulpa fructus GARDENIA JASMINOIDES ELLIS flos, fructus, radix GARRYA RACEMOSA RAMIREZ cortex, folium GAULTHERIA PROCUMBENS L. herba, folium GENTIANA LUTEA L. radix, rhizoma GELIDIUM AMANSII J.V. LAMOUROUX thallus GERANIUM MACULATUM L. herba, radix, rhizoma GERANIUM PRATENSE L. herba GERANIUM ROBERTIANUM L. herba c. floribus GERANIUM ROTUNDIFOLIUM L. herba GERANIUM SANGUINEUM L. herba GEUM RIVALE L. herba, radix, summitas, GEUM URBANUM L. herba, radix, summitas GINKGO BILOBA L. folium, bud, GLYCINE MAX (L.) MERR. semen, semen germinatus, GLYCYRRHIZA GLABRA L. radix, rhizoma GNAPHALIUM ULIGINOSUM A. RICH herba GOSSYPIUM HERBACEUM L. pilus, radix, semen GRACILARIA spp. thallus GRIFFONIA SIMPLICIFOLIA (DC.) BAILL. semen GRIFOLA FRONDOSA (DICKS.) GRAY (Maitake) sporophorum GRIFOLA UMBELLATA PERS. sporophorum GRINDELIA HUMILIS HOOK et AR. herba c. floribus GRINDELIA ROBUSTA NUTT. herba c. floribus GRINDELIA SQUARROSA DUN. herba c. floribus GUAJACUM OFFICINALE L. resina, lignum GUAJACUM SANCTUM L. resina, lignum GUAZUMA ULMIFOLIA LAMK. cortex, fructus GYMNEMA SYLVESTRE R. BR. radix, folium, latex GYNOSTEMMA PENTAFILLUM (THUNB.) MAKINO herba GYPSOPHILA PANICULATA L. radix GYPSOPHILA ROKEJEKA DEL. radix HAEMATOCOCCUS PLUVIALIS FLOTOW thallus HAMAMELIS VIRGINIANA L. cortex, folium HAPLOPAPPUS BAYLAHUEN REMY folium, herba HARPAGOPHYTUM PROCUMBENS DC. radix HARUNGANA MADAGASCARIENSIS LAM. ex POIRET cortex, folium HEDEOMA PULEGIOIDES PERS. folium, aetheroleum, herba c. floribus, summitas HEDERA HELIX L folium HEDICHIUM CORONARIUM KOENIG flos HELIANTHUS ANNUUS L. folium, ligula, semen HELIANTHUS TUBEROSUS L. tuber HELICHRYSUM ARENARIUM MOENCH. flos, herba c. floribus, summitas HELICHRYSUM ITALICUM DON. herba c. floribus, summitas HELICHRYSUM STOECHAS MOENCH. herba c. floribus, summitas HERACLEUM SPHONDYLIUM L. herba HERNIARIA GLABRA L. herba c. floribus, summitas HERNIARIA HIRSUTA L. herba c. floribus HESPERIS MATRONALIS L. herba c. floribus HIBISCUS SABDARIFFA L. flos HIERACIUM MURORUM L. herba HIERACIUM PILOSELLA L. herba HIEROCHLOE ODORATA WAHL. herba HIMANTHALIA ELONGATA (L.) S. GRAY thallus HIPPOPHAE RHAMNOIDES L. fructus, oleum HIZIKIA FUSIFORMIS (HARVEY) OKAMURA thallus HORDEUM spp. semen, semen germinatus, HUMULUS LUPULUS L. strobilus HYDRANGEA ARBORESCENS L. rhizoma HYDROCOTYLE VULGARIS L. herba HYGROPHILA AURICOLATA HEINE folium, radix, semen HYMENAEA COURBARIL L. cortex, resina HYPERICUM PERFORATUM L. flos, herba c. floribus, summitas, HYPTIS SUAVEOLENS POIT. herba c. floribus HYSSOPUS OFFICINALIS L. summitas, herba ILEX PARAGUARIENSIS A. St. HILL. (Matè) folium ILLICIUM VERUM HOOK.f. fructus, aetheroleum IMPATIENS NOLI-TANGERE L. herba INDIGOFERA TINCTORIA L. folium INULA CONYZA DC. herba INULA HELENIUM L. radix, rhizoma c. radicibus, folium IPOMEA BATATAS (L.) LAMB. tuber ISATIS TINCTORIA L. folium, herba, succus ISPAGULA (vedi PLANTAGO OVATA FORSK.) semen, tegumentum seminis JACARANDA CAROBA DC. flos, folium JASMINUM OFFICINALE L. flos JATEORHIZA PALMATA MIERS radix JUGLANS CINEREA L. cortex JUGLANS REGIA L. pericarpium, semen, folium, bud JUMELLEA FRAGRANS SCHLTR. folium JUNIPERUS COMMUNIS L. fructus, lignum, folium, aetheroleum, sùrculi (young shoots) JUSTICIA PECTORALIS JACQ. herba KAEMPFERIA GALANGA L. rhizoma KHAYA SENEGALENSIS JUSS cortex KICKXIA SPURIA DUMORT. herba c. floribus KNAUTIA ARVENSIS COULT. flos, folium KOLA ACUMINATA R. BR. semen KRAMERIA TRIANDRA RUIZ et P. radix LACHNANTHES TINCTORIA (WALTER ex J. F. GMEL.) ELLIOTT radix LACTUCA INDICA L. folium LACTUCA SATIVA L. folium LAGENARIA SICERARIA STANDL. semen LAGERSTROEMIA SPECIOSA (L.) PERS. (Banaba) folium LAMINARIA CLOUSTONI LE JOLY thallus LAMINARIA DIGITATA (HUDSON) J.V. LAMOUROUX (Alga Kombu) thallus LAMINARIA SACCHARINA (L.) LAMOUROUX (Alga Kombu) thallus, LAMIUM ALBUM L . flos, herba c.floribus LAPSANA COMMUNIS L. folium LARIX DECIDUA MILL. resina LASERPITIUM LATIFOLIUM L. herba, radix LASIOSPHAERA GIGANTEA BATCH ex PERS. sporophorum LAURUS NOBILIS L. fructus, folium, aetheroleum LAVANDULA x HYBRIDA REVERCHON flos, summitas c. floribus, aetheroleum LAVANDULA INTERMEDIA LOISEL flos, summitas c. floribus, aetheroleum LAVANDULA LATIFOLIA MEDIC. flos, summitas c. floribus, aetheroleum, herba LAVANDULA OFFICINALIS CHAIX (var.) flos, summitas c. floribus, aetheroleum LAVANDULA STOECHAS L. flos, summitas c. floribus, aetheroleum LAVANDULA VERA DC. flos, summitas c. floribus, aetheroleum LAWSONIA INERMIS L. folium LEDUM PALUSTRE L. herba LENS CULINARIS MEDIK. semen LENTINULA EDODES (BERK.) PEGLER (Shitake) sporophorum LEONOTIS NEPETIFOLIA AIT F. folium, herba c. floribus LEONURUS CARDIACA L. folium, herba c. floribus LEPIDIUM CAMPESTRE R. BR. semen, herba LEPIDIUM LATIFOLIUM L. herba LEPIDIUM MEYENII WALP. (Maca) radix, tuber LEPIDUM SATIVUM L. folium LEPTANDRA VIRGINICA NUTT. rhizoma LEPTOSPERMUM CITRATUM CHALL. summitas LEPTOSPERMUM SCOPARIUM J.R.FORST. & G.FORST. (MANUKA) folium, cortex, oleum LESPEDEZA CAPITATA MICH. folium, herba, summitas LEUCANTHEMUM VULGARE LAM. herba, capitula LEVISTICUM OFFICINALE KOCH. fructus, herba, folium, rhizoma, radix LIMONIUM VULGARE MILLER . radix, flos LINARIA VULGARIS MILL. herba c. floribus LINUM CATHARTICUM L. herba LINUM USITATISSIMUM L. semen, oleum, tegumen seminis LIPPIA CITRIODORA KUNTH flos, folium LIPPIA DULCIS TREV. flos, folium LIQUIDAMBAR ORIENTALIS MILL. balsamum, cortex LIRIODENDRON TULIPIFERA L. cortex LIRIOSOMA OVATA MIERS lignum LITCHI CHINENSIS SONNER semen LITHOSPERMUM OFFICINALE L. folium, radix, semen, fructus LITHOTHAMNIUM CALCAREUM thallus LITSEA CUBEBA PERS. fructus LOBARIA PULMONARIA HOFFM. thallus LONICERA JAPONICA THUNB. flos, stipites c. foliis et floribus LOTUS CORNICOLATUS L. herba, flos LUCUMA GLYCYPHLOEA MART-EIC. cortex LUPINUS spp semen, LYCIUM BARBARUM L. fructus LYCOPODIUM CLAVATUM L. herba, spora LYCOPUS EUROPAEUS L. herba LYCOPUS VIRGINICUS MICHX. herba LYSIMACHIA NUMMULARIA L. herba, radix LYSIMACHIA VULGARIS L. herba LYTHRUM SALICARIA L. summitas MACADAMIA TERNIFOLIA F. MUELL. fructus, semen MACROCYSTIS PYRIFERA (L.) C.AG. (Kelp) thallus MAGNOLIA OFFICINALIS REHDER cortex, flos MAHONIA AQUIFOLIUM PURSH. cortex, fructus, radix MAJORANA HORTENSIS MOENCH. folium, summitas, aetheroleum MALPIGHIA GLABRA L. (Syn. MALPIGHIA PUNICIFOLIA L. - Acerola) fructus MALUS DOMESTICA BORKH. (Syn. M. SYLVESTRIS (I.) Mill.) fructus, bud MALVA SYLVESTRIS L. flos, folium MAMMEA AMERICANA L. flos, fructus, folium, resina MANGIFERA INDICA L. fructus, folium, pericarpium MANIHOT ESCLUTENTA CRANTZ radix MARANTA ARUNDINACEA L. rhizoma MARCHANTIA POLYMORPHA L. thallus MARRUBIUM VOLGARE L. folium, herba c. floribus MARSDENIA CONDURANGO NICH. cortex arboris MARSDENIA REICHENBACHII TR. cortex arboris MATRICARIA CHAMOMILLA L. flos, herba, ligula, aetheroleum MEDICAGO SATIVA L. herba c. floribus MELALEUCA ALTERNIFOLIA CHEEL aetheroleum, summitas MELALEUCA LEUCADENDRON L. folium, aetheroleum, flos, cortex MELALEUCA LEUCADENDRON L. VAR.CAJAPUTI R. folium, aetheroleum, flos, cortex MELALEUCA LEUCADENDRON L. VAR.VIRIDIFLORA folium, aetheroleum MELALEUCA LINARIFOLIA SM. summitas MELILOTUS OFFICINALIS PALLAS flos, herba c. floribus, folium MELISSA OFFICINALIS L. folium, herba, aetheroleum MELITTIS MELISSOPHYLLUM L. folium, herba, flos MENTHA AQUATICA L. folium, summitas, aetheroleum MENTHA ARVENSIS L. folium, summitas, aetheroleum MENTHA x PIPERITA L. folium, summitas, aetheroleum MENTHA SPICATA L. folium, herba, summitas, aetheroleum MENTZELIA CORDIFOLIA DON. radix, summitas MENYANTHES TRIFOLIATA L. herba, folium MESPILUS GERMANICA L. fructus MEUM ATHAMANTICUM JACQ. radix MICHELIA CHAMPACA L. folium, aetheroleum, flos, cortex MIKANIA AMARA WILLD. herba, radix MIKANIA GUACO H. et B. herba MITCHELLA REPENS L. herba, MOMORDICA BALSAMICA L. fructus MOMORDICA CHARANTIA L. fructus, balsamum MONARDA PUNCTATA L. herba MORINDA CITRIFOLIA L. (NONI) succus, spuma (purea), fructus densatus (concentrato dei frutti), MORINDA OFFICINALIS HOW. herba MORINGA APTERA GAERTN. semen MORINGA OLEIFERA LAMK. semen, oleum MORRENIA BRACHYSTEPHANA GRIS. flos, radix, succus MORUS ALBA L cortex ex radicibus, folium, fructus, radix, ramulus MORUS NIGRA L. cortex ex radicibus, folium, fructus, bud MORUS spp. cortex ex radicibus, folium, fructus MUSA x PARADISIACA L. (pro spp.) fructus MYOSOTIS SCORPIOIDES L. flos, herba MYRCIARIA DUBIA (KUNTH) McVAUGH (Camu Camu) fructus MYRICA CERIFERA L. cortex, fructus MYRICA GALE L. folium, herba MYRISTICA FRAGRANS HOUTT. fructus, pericarpium, aetheroleum MYROXYLON BALSAMUM PEREIRAE KL. balsamum MYROXYLON BALSAMUM TOLUIFERUM H.B.K. balsamum MYRRHIS ODORATA SCOP. herba MYRTUS COMMUNIS L. summitas, folium, fructus, flos, aetheroleum NARDOSTACHYS JATAMANSI DC. folium, radix NASTURTIUM OFFICINALE R. BROWN herba NELUMBO NUCIFERA GAERTN. semen, radix NEPETA CATARIA L. herba c. floribus NEPHELIUM LAPPACEUM L. fructus sine semen NYMPHOIDES PELTATA KUNTZE herba OCIMUM BASILICUM L. folium OCIMUM GRATISSIMUM L. folium OCIMUM SANCTUM L. herba OENOTHERA BIENNIS L. semen, oleum OLEA EUROPAEA L. fructus, folium, sùrculi (young shoots), bud, oleum ex fructibus ONONIS ARVENSIS L. herba, radix ONONIS SPINOSA L. herba, radix ONOPORDON ACANTHIUM L. herba, radix ONOSMA ECHIOIDES L. s. l. folium, radix OPHIOGLOSSUM VULGATUM L. sarmentum, herba OPHIOPOGON JAPONICUS KER-GAW. radix tuberosa OPHIORRHIZA MUNGOS L. radix OPOPANAX CHIRONIUM KOCH. gummi, resina OPUNTIA FICUS-INDICA MILL. flos, cladodium, fructus ORCHIS MASCULA L. tuber, ORIGANUM DICTAMNUS L. flos, folium, herba c. floribus ORIGANUM VULGARE L. folium, summitas, aetheroleum ORTHOSIPHON STAMINEUS BENTH folium, herba c. floribus ORYZA SATIVA L. semen OSMUNDA REGALIS L. folium, rhizoma OXALIS ACETOSELLA L. folium OXYCOCCUS PALUSTRIS PERS. (Cranberry) fructus PACHIRA AQUATICA AUBL. semen, folium PACHIRA INSIGNIS SAV. folium PAEONIA LACTIFLORA PALL. semen, radix PAEONIA OFFICINALIS L. flos, radix PALMARIA PALMATA (L.) KUNTZE (Alga Dulse) thallus PANAX GINSENG C.A. MEYER folium, radix PANAX NOTOGINSENG CHEN. folium, radix PANAX PSEUDOGINSENG WALL. folium, radix PANAX QUINQUEFOLIUM L. folium, radix PANICUM MILIACEUM L. fructus PANZERIA LANATA BGE. herba PAPAVER RHOEAS L. flos, semen, folium PARIETARIA OFFICINALIS L . herba PARMELIA SAXATILIS ACH. thallus PARTHENIUM HYSTEROPHORUS L. herba PASSIFLORA EDULIS SIMS herba, fructus PASSIFLORA INCARNATA L. herba c. floribus PASTINACA SATIVA L. tuber, herba PAULLINIA CUPANA H.S.K. semen PEDALIUM MUREX L. fructus, semen, folium PEDICULARIS PALUSTRIS L. herba PEDILANTHUS PAVONIS BOISS. folium PELARGONIUM GRAVEOLENS L 'HERIT flos, folium PELARGONIUM RADULA L'HERIT flos, folium PERILLA FRUTESCENS BRITTON semen, oleum, folium PERSEA AMERICANA MILL. fructus PETIVERIA ALLIACEA L. folium, radix PETROSELINUM CRISPUM A.W.HIL. radix, folium, summitas, fructus PEUCEDANUM OFFICINALE L. radix PEUCEDANUM OREOSELINUM MOENC. radix PEUCEDANUM OSTRUTHIUM KOCH. rhizoma PEUMUS BOLDUS MOLINA cortex , folium PFAFFIA PANICULATA (MART.) KUNTZE radix PHASEOLUS VULGARIS L. fructus, semen PHILLYREA LATIFOLIA L. folium PHOENIX DACTYLIFERA L. fructus PHRAGMITES AUSTRALIS TRIN. radix PHYLLANTHUS AMARUS L. summitas PHYLLANTHUS EMBLICA L. fructus PHYLLANTHUS NIRURI L. folium, herba PHYLLITIS SCOLOPENDRIUM NEWM. folium, herba PHYSALIS ALKEKENGI L. fructus PHYSALIS PERUVIANA L. fructus PICRAMNIA ANTIDESMA SW. cortex PICRASMA EXCELSA PLANCH. cortex, lignum, PICRASMA QUASSIOIDES BENN. lignum PICRORHIZA KURROA ROYLE rhizoma PIMENTA DIOICA (L.) MERR. fructus PIMENTA RACEMOSA MOORE fructus, folium, aetheroleum PIMPINELLA ANISUM L. fructus, aetheroleum PIMPINELLA MAJOR HUDSON radix PIMPINELLA SAXIFRAGA L. herba, radix, rhizoma PINCKNEYA PUBENS MICHX. cortex PINUS KORAIENSIS SIEBOLD & ZUCC. semen, oleum PINUS MASSONIANA LAMB. semen, bud, folium, aetheroleum , cortex PINUS MUGO TURRA (syn. Pinus montana Mill.) bud, aetheroleum PINUS PINEA L. semen, cortex PINUS PINASTER AITON cortex, semen, bud, folium, aetheroleum PINUS SYLVESTRIS L. fructus, bud, folium, aetheroleum PIPER ANGUSTIFOLIUM RUIZ-PAV. folium PIPER CUBEBA L. fructus PIPER LONGUM L. fructus PIPER NIGRUM L. fructus, oleoresina, oleum PISTACHIA VERA L. fructus PISTACIA LENTISCUS L. resina, radix PISTACIA TEREBINTHUS L. cortex PISUM SATIVUM L. fructus, bacellus PITHECELLOBIUM AVAREMOTEMO MA. oleum PLANTAGO AFRA L. semen PLANTAGO ARENARIA POIRET semen PLANTAGO LANCEOLATA L. folium, herba c. floribus PLANTAGO MAJOR L. folium, herba, herba c. floribus, semen PLANTAGO MEDIA L. folium, herba PLANTAGO OVATA FORSK. (Ispagul) semen, tegumentum seminis PLANTAGO PSYLLIUM L. semen, tegumentum seminis PLATANUS ORIENTALIS L. bud PLECTRANTHUS BARBATUS ANDREWS (Syn. COLEUS FORSKOHLII) folium, radix, tuber PLEUROTUS OSTREATUS (JACQ. : FR.) P. KUMM sporophorum PLUMBAGO EUROPAEA L. herba, radix POLLINE POLYGALA AMARA L. herba c. floribus, radix POLYGALA VULGARIS L. s. l. herba, radix POLYGONUM AVICULARE L. herba POLYGONUM BISTORTA L. herba, radix, rhizoma POLYGONUM CUSPIDATUM SIEBOLD & ZUCC. radix POLYGONUM DUMETORUM L. herba POLYGONUM HYDROPIPER L. herba POLYGONUM MULTIFLORUM THUNB. cortex, rhizoma, radix POLYGONUM PERSICARIA L. folium, herba POLYPODIUM CALAGUALA RUIZ. radix, rhizoma POLYPODIUM LEUCOTOMOS L. summitas, rhizome, POLYTHRICUM COMMUNE L. herba POLYPORUS UMBELLATUS (PERS.: FR.) FR. (syn GRIFOLA UMBELLATA) sporophorum POPULUS ALBA L. cortex, bud, folium POPULUS BALSAMIFERA L. cortex, bud, folium POPULUS NIGRA L. cortex, bud, folium, lignum POPULUS TREMULOIDES L. cortex, folium, bud PORIA COCOS F. A. WOLF sclerotia PORPHYRA PALMATA (L.) K. (Alga Nori) thallus PORPHYRA TENERA KJELLMAN thallus PORPHYRA UMBILICALIS (L.) K. (Alga Nori) thallus PORTULACA OLERACEA L. herba, folium, radix POTENTILLA ANSERINA L. rhizoma, herbe c. floribus POTENTILLA ARGENTEA L. rhizoma, herba, herba c. floribus POTENTILLA ERECTA RAUSCHEL rhizoma, herba POTENTILLA REPTANS L. rhizoma POTERIUM SPINOSUM L. cortex ex pricipali radice PRANGOS PABULARIA LINDL. fructus PRIMULA ELIATOR SCHREB. flos, radix PRIMULA OFFICINALIS HILL. flos, radix PRIMULA VERIS L. flos, radix PRIMULA VULGARIS HUDS. flos, radix PROPOLIS resina PROTIUM GUIANENSE MARCH. resina PROTIUM HEPTAPHYLLUM MARCH. resina, lignum PROTIUM ICICARIBA MARCH. resina PRUNELLA VULGARIS L. folium, herba, flos PRUNUS ARMENIACA L. oleum PRUNUS CERASUS L. pedunculus PRUNUS DOMESTICA L. (syn. Sorbus domestica L.) fructus, bud PRUNUS DUILCIS PHILIP MILLER (syn. Prunus amygdalus Batsch) fructus, oleum, bud PRUNUS LAUROCERASUS L. semen, fructus PRUNUS MALAHEB L. semen PRUNUS PADUS L. cortex PRUNUS PERSICA BATSCH. cortex, flos, folium PRUNUS SEROTINA EHRH. cortex, folium PRUNUS SPINOSA L. flos, folium, fructus, succus fructi , bud PSEUDOWINTERA COLORATA (RAOUL) DANDY folium, cortex PSIDIUM ARACA RADDI. cortex, folium, fructus, radix PSIDIUM GUAJAVA L. folium PTEROCARPUS DRACO L resina PTEROCARPUS ERINACEUS POIR. succus inspissitus PTEROCARPUS INDICUS WILLD. lignum PTEROCARPUS MARSUPIUM ROXB. succus inspissitus, lignum PTEROCARPUS SANTALINUS L. f. lignum PTYCHOPETALUM OLACOIDES BENTH. (MUIRA PUAMA) cortex, cortex ex radicidus, lignum PUERARIA LOBATA (WILLD.) OHWI. rhizoma, radix PUERARIA TUBEROSA (ROXB. ex WILLD.) DC. Rhizoma, PULMONARIA OFFICINALIS L. folium, herba c. floribus PUNICA GRANATUM L. fructus, pericarpium, flos, semen PYROLA ROTUNDIFOLIA L. folium PYRUS COMMUNIS L. folium PYRUS SORBUS GAERTN. (syn. SORBUS DOMESTICA L.) bud QUASSIA AMARA L. lignum QUERCUS ALBA L. cortex, fructus, lignum QUERCUS COCCIFERA L. cortex QUERCUS ILEX L. cortex, fructus, lignum, sùrculi (young shoots) QUERCUS INFECTORIA OLIV. galla QUERCUS PETRAEA LIEBL. cortex, folium, semen QUERCUS PUBESCENS WILLD. cortex QUERCUS ROBUR L. cortex, folium, semen, lignum, galla, bud QUERCUS SUBER L . cortex QUILLAJA SAPONARIA MOL. cortex QUILLAJA SMEGMADERMOS DC. cortex RAPHANUS SATIVUS L. var. NIGER MILLER semen, radix RAPHANUS SATIVUS L. var. SATIVUS PERS. semen, radix RAPHIA PEDUNCULATA BEAUV. amylum RAVENSARA AROMATICA GMEL. fructus RHAMNUS ALPINUS L. cortex, fructus RHAMNUS ALPINUS L. SSP FALLAX M. et P. cortex, fructus RHAMNUS CATHARTICUS L. cortex, fructus immaturus RHAMNUS FRANGULA L. cortex RHAMNUS PURSHIANA DC cortex RHEMANNIA GLUTINOSA (GAERTN.) LIBOSCH. radix RHEUM EMODI WALL. radix, rhizoma RHEUM HYBRIDUM MURRAY radix, rhizoma RHEUM OFFICINALE BAILL. radix, rhizoma RHEUM PALMATUM L. var. TAGUNTICUM MAXIM radix, rhizoma RHEUM RHAPONTICUM L. radix, rhizoma RHEUM UNDULATUM L. radix, rhizoma RHODIOLA CRENULATA (HOOK. f. & THOMSON) H. OHBA radix RHODIOLA ROSEA L. radix RHODYMENIA PALMATA (L.) GREVILLE (Alga Dulse) thallus RHUS AROMATICA AITON cortex ex radicibus RHUS CORIARIA L. folium RHUS GLABRA L. fructus RIBES NIGRUM L. folium, fructus, flos, semen, oleum, bud RIBES RUBRUM L. folium RIBES UVA-CRISPA L. folium, fructus ROBINIA PSEUDACACIA L. flos ROCELLA TINCTORIA DC thallus ROHODODENDRON FERRUGINEUM L. folium ROSA CANINA L. fructus, rosae pseudofructus, sùrculi (young shoots), ROSA CENTIFOLIA L. flos, oleum ROSA GALLICA L. fructus, flos ROSA MOSCHATA HERM. Oleum, ROSMARINUS OFFICINALIS L. aetheroleum, folium, sùrculi (young shoots) ROYAL JELLY (Pappa reale) RUBIA CORDIFOLIA L. radix, rhizoma RUBUS CEASIUS L. fructus RUBUS FRUTICOSUS L.S. L. folium, fructus, sùrculi (young shoots) RUBUS IDAEUS L. folium, fructus, sùrculi (young shoots) RUBUS SUAVISSIMUS S. LEE folium, fructus RUMEX ACETOSA L. herba, radix, folium RUMEX ACETOSELLA L. herba, radix, folium RUMEX ACUTUS L. radix, folium RUMEX ALPINUS L. herba, radix, rhizoma RUMEX CRISPUS L folium, radix, rhizoma RUMEX LONGIFOLIUS DC. radix, folium RUMEX OBTUSIFOLIUS L. radix RUMEX PATIENTIA L. herba, radix RUMEX SANGUINEUS L. radix RUMEX SCUTATUS L. folium RUSCUS ACULEATUS L. radix, rhizoma RUSCUS HYPOGLOSSUM L. folium, rhizoma c. radicibus SABBATIA ANGULARI PURSH. herba SACCHARUM OFFICINARUM L. succus SAGERAEA LAURIFOLIA BLATTER folium SALACIA RETICULATA WIGHT radix SALIX ALBA L. cortex, folium, bud, cortex ramolorum (scorze dei giovani rami), flores (amenti), lympha, SALVIA LAVANDULIFOLIA VAHL aetheroleum SALVIA MILTIORRHIZA BGE. folium, radix SALVIA OFFICINALIS L. folium, aetheroleum SALVIA PRATENSIS L. folium, summitas c. floribus SALVIA SCLAREA L. flos, herba c. floribus SAMBUCUS CANADENSIS L. flos SAMBUCUS EBULUS L. flos SAMBUCUS NIGRA L. flos, cortex, folium, fructus SANGUISORBA MINOR SCOP. herba SANGUISORBA OFFICINALIS L. herba, radix SANICULA EUROPAEA L. folium, herba, radix SANTALUM ALBUM L. lignum, cortex, aetheroleum SAPONARIA OFFICINALIS L. folium, herba, radix SARRACENIA PURPUREA L. folium, rhizoma SATUREJA HORTENSIS L. summitas, semen SATUREJA MONTANA L. s. l. folium, herba c. floribus, aetheroleum SATUREJA THYMBRA L. herba c. floribus SAXIFRAGA GRANULATA L. folium, radix SCHINOPSIS QUEBRACHO COLORADO (SCHLTDL.) F.A. BARKLEY et T. MEYER lignum SCHINUS MOLLE L. folium, fructus, resina, SCHISANDRA CHINENSIS BAILL. folium, fructus SCORZONERA HISPANICA L. radix SCUTELLARIA BAICALENSIS GEORGI folium, radix SCUTELLARIA LATERIFLORA L. herba SECALE CEREALE L. fructus, radiculae SEDUM ACRE L. folium SEDUM ALBUM L. folium SEMPERVIVUM TECTORUM L. folium, herba SEQUOIADENDRON GIGANTEUM (LINDL.) J.BUCHH. bud, sùrculi (young shoots) SERENOA REPENS (BATRAM) SMALL. (Syn. SERENOA SERRULATA HOOK F.) fructus, SESAMUM INDICUM L. (Syn. SESAMUM ORIENTALE L.) semen SESELI TORTUOSUM L. fructus SHOREA WIESNERI SCHIFF. resina SIGESBECKIA ORIENTALIS L. folium, radix SILAUM SILAUS SHINZ ET THELL. radix, fructus SILYBUM MARIANUM GAERTN. fructus, tegumen seminis, herba SIMABA CEDRON PLANCH. semen SIMAROUBA AMARA AUBL. cortex SIMMONDSIA CHINENSIS NUTT. oleum SISON AMOMUM L. fructus SISYMBRIUM OFFICINALE SCOP. folium, summitas c. floribus SIUM ERECTUM HUDS. herba SIUM LATIFOLIUM L. fructus, herba SIUM NINZI L. radix SIUM SISARUM L. herba, radix SMILAX ARISTOLOCHIAEFOLIA MILLER radix SMILAX ASPERA L. radix SMILAX CHINA L . radix SMILAX FEBRIFUGA KUNTH. radix SMILAX MEDICA SCHLECHT et C. radix SMILAX OFFICINALIS H.B.K. radix SMILAX ORNATA LAMK . radix SMILAX REGELII KILLIP et MOR. radix SMILAX UTILIS HEMSL. radix SOLANUM LYCOPERSICUM L. fructus SOLANUM MELONGENA L. fructus SOLANUM TUBEROSUM L. tuber SOLIDAGO VIRGA-AUREA L. herba c. floribus SORBUS AUCUPARIA L. semen, fructus SORGHUM VULGARE PERS. semen SORGHUM BICOLOR (L.) MOENCH semen SPATHYEMA FOETIDA RAF. herba, radix, rhizoma SPERGULARIA RUBRA J. et PRESL. herba c. floribus SPILANTHES ACMELLA MURR. var. OLERACEA L. herba c. floribus SPINACIA OLERACEA L. folium SPIRULINA MAXIMA thallus SPIRULINA PLATENSIS thallus SPILANTHES ACMELLA MURR. capitula, herba, folium, STACHYS OFFICINALIS (L.) TREVIS. (syn. BETONICA OFFICINALIS L.) folium, herba, summitas STACHYS RECTA L. herba STACHYS SYLVATICA L. herba STELLARIA MEDIA VILL. herba STERCULIA URENS ROXB. gummi STYRAX BENZOIDES CRAIB. balsamum, resina STYRAX BENZOIN DRYANDER balsamum STYRAX OFFICINALIS L. balsamum STYRAX PARALLELONEURUM PERK. balsamum STYRAX TONKINENSIS CRAIS. balsamum SUCCISA PRATENSIS MOENCH. flos, folium SWERTIA CHIRATA BUCH. HAM. herba, radix SWERTIA JAPONICA MAKINO herba, radix SYRINGA VULGARIS L. flos, folium, fructus, cortex, bud SYZYGIUM AROMATICUM (L.) MERR. et L. M. PERRY. (syn. EUGENIA CARYOPHYLLATA THUNB.) fructus, flos, folium, aetheroleum SYZYGIUM CUMINI SKEELS folium, fructus, cortex, semen SYZYGIUM JAMBOS ALSTON flos, folium, fructus, cortex, semen SYZYGIUM MALACCENSE MERR. et L. M. PERRY. flos, folium, cortex TABEBUIA AVELLANEDAE LORENTZ ex GRISEB. cortex, flos TAGETES ERECTA L. capitula TAGETES MINUTA L. flos, semen TAMARINDUS INDICA L. fructus, pulpa fructi, succus TAMARIX GALLICA L. cortex, galla, bud, sùrculi (young shoots) TANACETUM PARTHENIUM SCH. BIP. capitula, herba c. floribus TANACETUM VULGARE L. summitas, capitula, herba c. floribus TARAXACUM OFFICINALE WEBER herba c. radicibus, radix, folium TERMINALIA BELLERICA ROXB. fructus TERMINALIA CHEBULA RETZ. fructus TERMINALIA CITRINA ROXB. fructus THALICTRUM FLAVUM L. radix THEOBROMA CACAO L. semen THLASPI ARVENSE L. semen THYMUS SERPYLLUM L. s.l. folium, summitas, oleum THYMUS VULGARIS L. folium, summitas, oleum TILIA AMERICANA L. cortex TILIA CORDATA MILL. folium, flos, bud TILIA PLATYPHYLLOS SCOP. folium, flos, cortex, bud TILIA TOMENTOSA MOENCH flos, bud TRACHYSPERMUM AMMI SPRAGUE aetheroleum, fructus, semen TRADESCANTIA ELONGATA GFW M. folium TRAGOPOGON PORRIFOLIUS L. (Salsefrica) radix TRAMETES SUAVEOLENS FR. sporophorum TRIBULUS TERRESTRIS L. fructus, herba TRICHILIA CATIGUA A. JUSS. (Catuaba) cortex TRIDAX PROCUMBENS L. herba TRIFOLIUM PRATENSE L. flos, herba TRIGONELLA CAERULEA SER. folium, herba c. floribus, TRIGONELLA FOENUM-GRAECUM L. semen TRILISA ODORATISSIMA CASS. folium TRILLIUM ERECTUM L. rhizoma TRITICUM AESTIVUM L. fructus, oleum germinis (Syn. TRITICUM SATIVUM LAM.) TRITICUM DICOCCON SCHRANK. fructus TROPAEOLUM MAJUS L. fructus, herba, folium, semen TROPAEOLUM MINUS L. fructus, herba TSUGA CANADENSIS CARR. cortex, folium, resina, summitas TURNERA DIFFUSA WILLD. flos, folium, summitas ULMUS CAMPESTRIS L. cortex ULMUS FULVA MICHX. cortex, folium ULMUS MINOR MILLER cortex, flos, folium, bud ULMUS RUBRA MUHL. cortex ULVA spp. thallus UMBILICUS ERECTUS DC. folium, herba UMBILICUS HORIZONTALIS DC. folium, herba UMBILICUS RUPESTRIS DANDY folium, herba UNCARIA GAMBIR ROXB. folium, summitas UNCARIA RHYNCOPHYLLA MIQ. succus UNCARIA TOMENTOSA WILLD. (ex SCHULT.) DC. radix, cortex UNDARIA PINNATIFIDA (HARVEY) SURINGAR (Alga wakamè) thallus URTICA DIOICA L. folium, summitas, radix URTICA URENS L. folium, summitas, radix USNEA BARBATA L. s. l. thallus USNEA PLICATA WIGGERS thallus UVA SPINA (vedi RIBES UVA-CRISPA L.) folium, fructus VACCINIUM CORYMBOSUM L. fructus VACCINIUM MACROCARPUM AITON (Cranberry) folium, fructus VACCINIUM MYRTILLUS L. folium, fructus, sùrculi (young shoots) VACCINIUM ULIGINOSUM L. folium, fructus VACCINIUM VITIS-IDAEA L. flos, folium, sùrculi (young shoots) VALERIANA CELTICA L. radix VALERIANA OFFICINALIS L. radix, rhizoma VALERIANA OFFICINALIS L. ssp. SAMBUCIFOLIA radix VALERIANA PHU L. radix VALERIANELLA LOCUSTA L. folium VANILLA FRAGRANS (SALIS.) AMES fructus, oleoresina, extractum fluidum, extractum siccum VANILLA PLANIFOLIA ANDREWS fructus, oleoresina, extractum fluidum, extractum siccum VATERIA INDICA L. resina VERBASCUM DENSIFLORUM BERT. flos, folium, herba c. floribus VERBASCUM NIGRUM L. flos, folium VERBASCUM PHLOMOIDES L. flos, folium, herba c. floribus VERBASCUM THAPSUS L. flos, folium, herba c. floribus, VERBENA OFFICINALIS L. herba c. floribus VERONICA ALLIONI F.W. SCHMIDT herba VERONICA ANAGALLIS-AQUATICA L. folium VERONICA AUSTRIACA L. ssp. TEUCRIUM WEBB herba c. floribus VERONICA BECCABUNGA L. folium VERONICA CHAMAEDRYS L. herba c. floribus VERONICA OFFICINALIS L. herba c. floribus VETIVERIA ZIZANIOIDES NASH. radix VICIA ERVILIA WILLD. semen VICIA FABA L. herba c. floribus, flos, fructus VIOLA CANINA L . flos VIOLA ODORATA L. flos, folium, rhizoma VIOLA TRICOLOR L. s. l. flos, herba c. floribus VISCUM ALBUM L. folium, herba, sùrculi (young shoots) VITEX AGNUS-CASTUS L. folium, fructus, radix VITIS VINIFERA L. folium, fructus, semen, oleum, bud WASABIA JAPONICA (MIQ.) MATSUM (syn. EUTREMA JAPONICA (MIQ.) KOIDZ) radix, hypogaeos caulis WITHANIA SOMNIFERA (L.) DUNAL. radix XERANTHEMUM ANNUUM L. flos YUCCA FILAMENTOSA L. herba YUCCA SCHIDIGERA ROEZL ex ORTGIES folium, herba ZANTHOXYLUM ACANTHOPODIUM DC. cortex, fructus ZANTHOXYLUM AMERICANUM MILL. cortex, fructus ZANTHOXYLUM ARMATUM DC. cortex, fructus ZANTHOXYLUM PIPERITUM (L.) DC. fructus ZEA MAYS L. stigmata ZEA MAYS L. var. SACCHARATA (STURTEV.) L. H. BAILEY fructus, radix ZINGIBER OFFICINALIS ROSC. rhizoma, oleum ZIZIPHUS JUJUBA MILLER cortex, folium, fructus, semen.

Said functional active ingredient can also be selected from probiotics and prebiotics.

Said formulation is processed in punch presses, by way of example using compression in the 20 punch Ronchi PA automatic rotary tablet press.

The formulation according to the present invention advantageously offers the possibility of having tablets with the desired properties of water-solubility, properties that are observed both when the water is at room temperature and when the water is hot. The tablets are particularly useful for delivering functional active ingredients.

### Examples

Example 1: Dissolution of tablets according to the present invention, comparison with tablets of the prior art. Tablets having the composition shown in Table 1 have been prepared:

**Table 1**

| **INGREDIENT** | **CONCENTRATION** |
|---|---|
| MANNITOL | 21.34% |
| POLYDEXTROSE | 4.02% |
| HYDROXYPROPYL METHYLCELLULOSE | 2.44% |
| POLYVINYLPYRROLIDONE | 2.44% |
| COMPRITOL E ATO | 1.95% |
| MICRO CELLULOSE | 9.76% |
| SUCRALOSE | 0.49% |
| SILICON DIOXIDE | 2.44% |
| MAGNESIUM SALTS OF FATTY ACIDS | 1.71% |
| CROSPOVIDONE | 17.07% |
| BEET JUICE | 3.05% |
| RED FRUIT AROMA | 6.10% |
| CITRIC ACID | 8.90% |
| MALTODEXTRINS | 18.29% |

The tablets are obtained with a punch from the Lurga (P) company through compression in the 20 punch Ronchi PA automatic rotary tablet press and show the following specifications (Table 2):

**Table 2**

| **SPECIFICATIONS** | **VALUE** |
|---|---|
| Hardness | 25 - 35 N |
| Friability | 0.7-1.6 % |
| Thickness | 3.8-4.1 mm |
| Weight | 790 - 830 mg |

Tablets thus obtained according to the present invention have been compared for their dissolution ability with tablets available in the art, which make use of different excipients. In particular, the comparison was performed with the following samples:
Sample 1 (comparative): Multivix comprising: Dehydrated citric acid; Sodium bicarbonate; Maltodextrin; Magnesium oxide; lemon and limes; Ascorbic acid; Aspartame, Acesulfame K and Sorbitol; DL-alpha-tocopherol acetate; Nicotinamide; Iron sulphate monohydrate; palmitate retinol; Zinc sulphate monohydrate; calcium d-pantothenate; cholecalciferol; Riboflavin 5' sodium phosphate dihydrate; Pyridoxine hydrochloride; Manganese sulphate monohydrate; Thiamine hydrochloride; Copper sulphate pentahydrate; Folic acid; Chromium chloride hexahydrate; Biotin; Potassium iodide; Sodium Selenate; Sodium molybdate dihydrate; Cyanocobalamin.
Sample 2 (comparative): Zimox 1 g comprising: active ingredient: 1.148 grams of amoxicillin trihydrate, excipients: sodium carboxymethyl starch, colloidal silica, magnesium stearate, cellulose microcrystalline.
Sample 3 (comparative): O.R.S comprising: Dextrose (Glucose); Citric acid, sodium bicarbonate; Potassium chloride; Sodium chloride; Natural lemon flavor (Maltodextrin, Acacia Gum, Flavoring preparations); Cornstarch; Sucralose; Riboflavin.
Sample 4 (according to the invention): the formulation referred to in table 1.

For each of the samples, data on hardness (N), thickness (mm), friability (%), diameter (mm) and weight (mg) were collected on 10 tablets for each reference.

The methods used refer to the ISO 9001: 2015 quality management system.

To measure diameter, thickness and hardness a GLOBOPHARMA S.A.S. durometer was used.

An ERWEKA TA crusher was used to measure friability. The measurement is the result of an average of three tests, each performed on 10 tablets, for each sample. Each test consists of 100 rotations equal to an estimated time of 4 minutes.

To measure the weight, a RADWAG technical scale, model PS1000.R2, was used.

To evaluate the dissolution a magnetic stirrer with a VELP SCIENTIFICA heating plate, a 250 ml SIMAX beaker, a PLASTILAB KARTELL 3 cm long stirring bar, a COLORCON thermometer, deionized water using a HYDROTECHNICAL demineralizer with conductivity equal to 0.1 microsiemens and pH 5.5 ± 0.2 and HANHART chronograph model DELTA E 200, were used.

The experiment consists in solubilizing a sample of 10 tablets per reference, each in 100 ml of deionized water. The water was kept stirring by means of a stirring bar at 700 rpm and at a constant temperature. The dissolution rate of each tablet was measured by a chronograph.

Complessively the experiment was carried out at three different temperatures: 25 °C ± 2; 50 °C ± 2; 70 °C ± 2.

The results are summarized herein below ("*cpr*" *indicates tablet*):
**Sample 1 (comparative):**

| Parameter | **cpr 1** | **cpr 2** | **cpr 3** | **cpr 4** | **cpr 5** | **cpr 6** | **cpr 7** | **cpr 8** | **cpr 9** | **cpr 10** |
|---|---|---|---|---|---|---|---|---|---|---|
| Hardness (N) | 79 | 79,2 | 80 | 78,4 | 83,4 | 78,5 | 79,4 | 79,3 | 80,2 | 78,9 |
| Thickness (mm) | 6,01 | 6,03 | 6,25 | 5,89 | 6,03 | 6 | 5,95 | 6,02 | 5,89 | 6,02 |
| Weight (mg) | 4497 | 4508 | 4524 | 4480 | 4492 | 4520 | 4500 | 4495 | 4509 | 4486 |
| Diameter (mm) | 25,36 | 25,36 | 25,34 | 25,35 | 25,36 | 25,33 | 25,35 | 25,34 | 25,36 | 25,33 |

| Parameter | Series 1 (10 cpr) | Series 2 (10 cpr) | Series 3 (10 cpr) |
|---|---|---|---|
| Friability (%) | 16,23 | 13,45 | 15,04 |

**Sample 2 (comparative):**

| Parameter | **cpr 1** | **cpr 2** | **cpr 3** | **cpr 4** | **cpr 5** | **cpr 6** | **cpr 7** | **cpr 8** | **cpr 9** | **cpr 10** |
|---|---|---|---|---|---|---|---|---|---|---|
| Hardness (N) | 83,3 | 80,5 | 85,6 | 82,4 | 84,6 | 81,2 | 81,7 | 83,7 | 84,4 | 82,4 |
| Thickness (mm) | 8 | 7,95 | 8 | 7,97 | 8,02 | 7,97 | 7,95 | 7,98 | 8 | 7,95 |
| Weight (mg) | 1439 | 1442 | 1437 | 1438 | 1445 | 1440 | 1439 | 1437 | 1448 | 1440 |
| Diameter (mm) | 21,52 | 21,5 | 21,48 | 21,52 | 21,52 | 21,51 | 21,47 | 21,45 | 21,5 | 21,48 |

| Parameter | Series 1 (10 cpr) | Series 2 (10 cpr) | Series 3 (10 cpr) |
|---|---|---|---|
| Friability (%) | 1,02 | 1,23 | 0,98 |

**Sample 3 (comparative):**

| Parameter | **cpr 1** | **cpr 2** | **cpr 3** | **cpr 4** | **cpr 5** | **cpr 6** | **cpr 7** | **cpr 8** | **cpr 9** | **cpr 10** |
|---|---|---|---|---|---|---|---|---|---|---|
| Hardness (N) | 98,6 | 76,7 | 73,1 | 72,8 | 46,2 | 71,2 | 71,7 | 72,5 | 70,4 | 71,9 |
| Thickness (mm) | 4,57 | 4,56 | 4,65 | 4,61 | 4,65 | 4,65 | 4,62 | 4,61 | 4,65 | 4,59 |
| Weight (mg) | 2,94 | 2,96 | 2,96 | 2,97 | 2,97 | 2,96 | 2,96 | 2,97 | 2,96 | 2,95 |
| Diameter (mm) | 25,24 | 25,2 | 25,23 | 25,24 | 25,29 | 25,26 | 25,26 | 25,27 | 25,26 | 25,23 |

| Parameter | Series 1 (10 cpr) | Series 2 (10 cpr) | Series 3 (10 cpr) |
|---|---|---|---|
| Friability (%) | 3,31 | 4,03 | 2,8 |

**Sample 4 (Velotransfer, according to the invention):**

| Parameter | **cpr 1** | **cpr 2** | **cpr 3** | **cpr 4** | **cpr 5** | **cpr 6** | **cpr 7** | **cpr 8** | **cpr 9** | **cpr 10** |
|---|---|---|---|---|---|---|---|---|---|---|
| Hardness (N) | 27,2 | 28,2 | 38,7 | 33,1 | 33,5 | 32,1 | 33,8 | 28,9 | 29,2 | 33,8 |
| Thickness (mm) | 3,91 | 3,88 | 3,92 | 3,91 | 3,94 | 3,92 | 3,9 | 3,92 | 3,84 | 3,9 |
| Weight (mg) | 805 | 801 | 823 | 790 | 805 | 812 | 800 | 825 | 811 | 810 |
| Diameter (mm) | 16,29 | 16,32 | 16,32 | 16,32 | 16,35 | 16,34 | 16,32 | 16,32 | 16,37 | 16,36 |

| Parameter | Series 1 (10 cpr) | series 2 (10 cpr) | Series 3 (10 cpr) |
|---|---|---|---|
| Friability (%) | 0,82 | 1,32 | 1,51 |

The dissolution rate at 25°C±2, 50°C±2, 75°C±2 is summarized in tables 3, 4, 5, respectively:

**Table 3: 25°C±2**

| | **Cpr1** | **Cpr2** | **Cpr3** | **Cpr4** | **Cpr5** | **Cpr6** | **Cpr7** | **Cpr8** | **Cpr9** | **Cpr10** | **Average** | **SD** |
|---|---|---|---|---|---|---|---|---|---|---|---|---|
| **1 (s)** | 83 | 73 | 70 | 72 | 73 | 68 | 71 | 69 | 74 | 79 | 73,20 | 4,61 |
| **2 (s)** | 21 | 20 | 23 | 23 | 24 | 24 | 24 | 25 | 25 | 20 | 22,50 | 1,84 |
| **3 (s)** | 80 | 74 | 82 | 81 | 72 | 81 | 64 | 72 | 72 | 71 | 74,90 | 5,88 |
| **4 (s)** | 68 | 58 | 45 | 50 | 62 | 56 | 59 | 66 | 54 | 58 | 57,60 | 6,93 |

**Table 4: 50°C±2**

| | **Cpr1** | **Cpr2** | **Cpr3** | **Cpr4** | **Cpr5** | **Cpr6** | **Cpr7** | **Cpr8** | **Cpr9** | **Cpr10** | **Average** | **SD** |
|---|---|---|---|---|---|---|---|---|---|---|---|---|
| **1 (s)** | 38 | 35 | 30 | 30 | 31 | 32 | 29 | 32 | 37 | 33 | 32,70 | 3,06 |
| **2 (s)** | 21 | 21 | 25 | 26 | 26 | 28 | 24 | 23 | 25 | 20 | 23,90 | 2,60 |
| **3 (s)** | 17 | 18 | 20 | 20 | 19 | 23 | 22 | 23 | 22 | 22 | 20,60 | 2,12 |
| **4 (s)** | 19 | 20 | 13 | 15 | 19 | 19 | 17 | 13 | 16 | 17 | 16,80 | 2,53 |

**Table 5: 75°C±2**

| | **Cpr1** | **Cpr2** | **Cpr3** | **Cpr4** | **Cpr5** | **Cpr6** | **Cpr7** | **Cpr8** | **Cpr9** | **Cpr10** | **Average** | **SD** |
|---|---|---|---|---|---|---|---|---|---|---|---|---|
| **1 (s)** | 17 | 14 | 16 | 16 | 18 | 17 | 12 | 17 | 15 | 14 | 15,60 | 1,84 |
| **2 (s)** | 19 | 19 | 20 | 21 | 15 | 18 | 17 | 22 | 18 | 17 | 18,60 | 2,07 |
| **3 (s)** | 16 | 16 | 14 | 16 | 13 | 14 | 15 | 14 | 14 | 15 | 14,70 | 1,06 |
| **4 (s)** | 10 | 11 | 9 | 9 | 9 | 10 | 10 | 9 | 8 | 10 | 9,50 | 0,85 |

The data obtained show how the formulation according to the present invention is surprisingly capable of leading to the desired dissolution results at each of the temperatures tested.

## Claims

1. Formulation in tablet form comprising:
- At least one functional active ingredient, up to 21% (w / w);
- At least one sweetener, selected from mannitol, xylitol, glucose, sucrose, saccharin, aspartame, sucralose, polydextrose, preferably mannitol 19-24% (w / w) and / or sucralose 0.3-0.6% (w / w) and / or polydextrose 3-6% (w / w);
- At least one binder, selected from from gelatins and cellulose derivatives, preferably selected from hydroxypropyl methylcellulose (HPMC), microcrystalline cellulose, arabic gum, glucose syrup and polyethylene glycols, preferably hydroxypropyl methylcellulose 1-5% (w / w) and microcrystalline cellulose 8-13% (w / w);
- At least one disintegrant, selected from maize starch, modified microcrystalline celluloses, polyvinylpyrrolidones, preferably polyvinylpyrrolidone 1-5% (w / w) and / or crospovidone 16-21 % (w / w)
- At least one glidant, selected from the magnesium salts of fatty acids 1-5% (w / w);
- At least one anti-caking agent, selected from carbonates, chlorides, silicon dioxide, preferably 1-5% silicon dioxide (w / w);
- At least one preservative, preferably 6-10% citric acid (w / w).

2. Formulation according to claim 1 comprising:
- At least one functional active ingredient, up to 21 % (w / w);
- Mannitol 19-24% (w / w)
- Polydextrose 3-6% (w / w)
- Hydroxypropyl methylcellulose 1-5% (w / w)
- Polyvinylpyrrolidone 1-5% (w / w)
- Microcrystalline cellulose 8-13% (w / w)
- Sucralose 0.3-0.6% (w / w)
- Silicon dioxide 1-5% (w / w)
- Magnesium salts of fatty acids 1-5% (w / w)
- Crospovidone 16-21% (w / w)
- Citric acid 6-10% (w / w).

3. Formulation according to claims 1 or 2, which comprises:
- At least one functional active ingredient, up to 21% (w / w);
- Mannitol 20-23% (w / w)
- Polydextrose 3.5-5% (w / w)
- Hydroxypropyl methylcellulose 2-4% (w / w)
- Polyvinylpyrrolidone 2-4% (w / w)
- Microcrystalline cellulose 9-11% (w / w)
- Sucralose 0.4-0.5% (w / w)
- Silicon dioxide 2-4% (w / w)
- Magnesium salts of fatty acids 1-3% (w / w)
- Crospovidone 16.5-19% (w / w)
- Citric acid 7-9.2% (w / w).

4. The formulation according to one of claims 1 to 3, wherein said functional active ingredient is between 16 and 21% (w / w).

5. The formulation according to one of claims 1 to 4, further comprising natural or artificial flavors, preferably in a range between 4-8% (w / w).

6. Formulation according to one of claims 1 to 5, comprising:
- At least one functional active ingredient, up to 21% (w / w);
- Mannitol 21.34% (w / w)
- Polydextrose 4.02% (w / w)
- Hydroxypropyl methylcellulose 2.44% (w / w)
- Polyvinylpyrrolidone 2.44% (w / w)
- Microcrystalline cellulose 9.76% (w / w)
- Sucralose 0.49% (w / w)
- Silicon dioxide 2.44% (w / w)
- Magnesium salts of fatty acids 1.71% (w / w)
- Crospovidone 17.07% (w / w)
- Citric acid 8.90% (w / w).

7. Formulation according to claim 6, further comprising:
- Glyceryl behenate E471 (Compritol ® E ATO) 1.95% (w / w)
- Beet juice 3.05% (w / w)
- Red fruit aroma 3.05% (w / w)

8. Formulation according to one of claims 1 to 7, wherein said functional active ingredient is selected from plant and / or animal extracts, mineral salts, vitamins, amino acids, proteins.

9. A food supplement formulated as a tablet which is formulated according to one of claims 1 to 8.
